# EUROPEAN PATENT SPECIFICATION

(11) **EP 2 415 393 B1**
(45) Date of publication and mention of the grant of the patent: **27.07.2016**
(21) Application number: 11176582.2
(22) Date of filing: 04.08.2011
(51) Int. Cl.: A61B 3/10

(54) **Ophthalmic apparatus**
Ophthalmische Vorrichtung
Appareil ophtalmique

(30) Priority: 05.08.2010 JP 2010176673; 27.06.2011 JP 2011141958
(43) Date of publication of application: 08.02.2012
(73) Proprietor: Nidek Co., Ltd., Gamagori-shi, Aichi 443-0038 (JP)
(72) Inventor: Endo, Masakazu, Okazaki-shi, Aichi 444-3513 (JP); Hanebuchi, Masaaki, Nukata-gun, Aichi 444-0103 (JP); Ochi, Hisashi, Gamagori-shi, Aichi 443-0035 (JP); Suzuki Kunio, Nagoya-shi, Aichi 456-0032 (JP)
(74) Representative: Zimmermann, Tankred Klaus

(56) References cited:
- EP-A2- 0 850 614
- WO-A1-00/56204
- WO-A1-2010/037485
- WO-A1-2010/080576
- DE-A1-102007 046 507
- US-A1- 2008 100 802

## Description

### BACKGROUND

### 1. Technical Field

An aspect of the present invention relates to an ophthalmic apparatus (ophthalmic measurement apparatus) for measuring an examinee's eye.

### 2. Related Art

Ophthalmic apparatuses have been known to measure an examinee's eye by projecting a measurement light flux onto a fundus of the eye and receiving the light reflected thereby. Such ophthalmic apparatuses are, for example, auto-refractometers and optical interference ocular axial length measuring apparatuses.

Among known auto-refractometers, there is an apparatus capable of capturing an image of an inner pupil (so called a retro-illumination image) by illuminating the inner pupil with fundus reflection light (see JP-A-8-112254). Such an apparatus can check an opacity state of a crystalline lens.

The ocular axial length measuring apparatus has an interference optical system to measure an ocular axial length of an examinee's eye (see, for example, JP-A-8-112254 stated above). The interference optical system irradiates the eye with measurement light and detects the light reflected thereby as interference light in a light receiving device.

EP 0 850 614 A2 describes an ophthalmic operating apparatus comprising an observation optical system for observing an anterior part of patient's eye, a laser beam irradiating optical system for irradiating a laser beam for treatment on the patient's eye, a moving device for moving the laser beam irradiating optical system relatively with respect to the patient's eye, an illumination optical system for projecting an illumination light from a pupil of the patient's eye so that the anterior part being illuminated by a reflected light at a fundus of the patient's eye from a rear side, and a photographic element for photographing a retro-illumination image of the anterior part illuminated by the illumination optical system.

Document US-A-2008/0100802 relates to an ophthalmic measurement apparatus for measuring wavefront aberration of an examiner's eye comprising speckle suppressing means.

### SUMMARY

It is an object of the present invention to provide an ophthalmic apparatus capable of obtaining a good retro-illumination image.

This object is achieved by an ophthalmic apparatus according to claim 1.

The present inventors used a light source (e.g., super luminescent diode: SLD), which emits coherent light, as a measurement light source, and captured a retro-illumination image. The result demonstrated that uneven spots (speckle noise) were generated across the image due to scattering of the light by a fundus. In such a case, an examiner is unable to distinguish between opacity caused by cataract and the uneven spots, causing difficulty in checking an opacity state.

Moreover, when the ocular axial length measuring apparatus in the related art is used to measure an ocular axial length of a severe cataract eye, measurement light is widely scattered by an opacity area of cataract. This causes a significant decrease in S/N ratio of an interference signal. Consequently, there are cases where an ocular axial length cannot be measured. The S/N ratio of the interference signal can also be decreased when a measurement optical axis is separated from a corneal apex. Accordingly, when an unskilled examiner uses the ocular axial length measuring apparatus, an adequate interference signal may not be obtained, or a measurement error may occur.

The ophthalmic apparatus of one aspect of the present invention includes the following configuration to achieve the object.

An ophthalmic apparatus for measuring an examinee's eye based on an output from a light receiving device includes a measuring optical system for measuring the examinee's eye, including: a light source for emitting light having coherence; a light projecting optical system for projecting measurement light, which is at least part of the light emitted from the light source, onto a fundus of the examinee's eye; and a light receiving optical system for guiding light including the measurement light reflected by the fundus of the examinee's eye to the light receiving device, characterized by an imaging optical system, including an imaging device having an imaging surface arranged in a position substantially conjugate with an anterior segment of the examinee's eye, for capturing a retro-illumination image using the imaging device by illuminating an inner pupil from behind with the measurement light reflected by the fundus, and a speckle suppressing means, arranged in an optical path of the light projecting optical system, for optically suppressing a speckle noise generated on the retro-illumination image.

According to such a configuration, the good retro-illumination image can be obtained.

### BRIEF DESCRIPTION OF THE DRAWINGS

Fig. 1 is a schematic diagram illustrating an ophthalmic apparatus according to a first embodiment of the present invention;
Fig. 2 is a schematic diagram illustrating the configuration of an optical system of the ophthalmic apparatus according to the first embodiment;
Fig. 3 is a diagram illustrating an anterior segment observation screen displaying an anterior segment image of an eye;
Fig. 4 is a diagram illustrating a retro-illumination image observation screen displaying a retro-illumination mage;
Fig. 5 is a schematic diagram illustrating the configuration of an optical system and a control system in an ophthalmic apparatus (eye refractive power measuring apparatuses) according to a second embodiment of the present invention;
Fig. 6A is a diagram illustrating a retro-illumination image observation screen before a corneal apex and an imaging optical axis are displaced;
Fig. 6B is a diagram illustrating a retro-illumination image observation screen after the corneal apex and the imaging optical axis are displaced;
Fig. 7 is a diagram explaining a method of detecting a displacement between a corneal apex position and a reference position;
Fig. 8 is a flowchart illustrating an exemplary procedure for improving a measurable rate of measurement of an examinee's eye;
Fig. 9 is a diagram illustrating partitioned regions within a measurable region of a retro-illumination image picture;
Fig. 10 is a diagram illustrating an exemplary screen on a monitor displaying a retro-illumination image picture and the partitioned regions with respective opacity rates calculated; and
Fig. 11 is a diagram explaining an arrangement of a measurement optical axis in a predetermined position of a partitioned region having a low opacity rate.

### DESCRIPTION OF EMBODIMENTS

In the following detailed description, for purpose of explanation, numerous specific details are set forth in order to provide a thorough understanding of the disclosed embodiments. It will be apparent, however, that one or more embodiments may be practiced without these specific details. In other instances, well-known structures and devices are schematically shown in order to simplify the drawing.

### <First Embodiment>

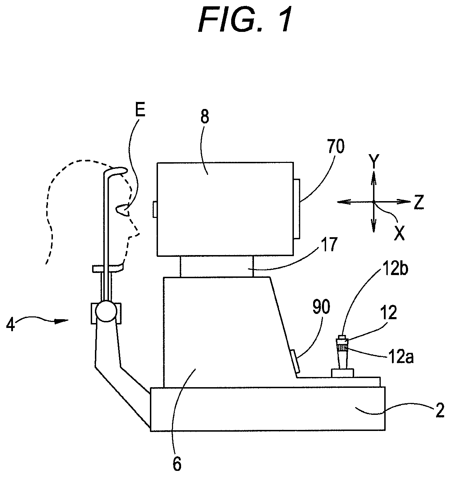

A first embodiment of the present invention will be described based on the accompanying drawings. Fig. 1 is a schematic diagram illustrating an ophthalmic apparatus (first ophthalmic apparatus) of the first embodiment. The first ophthalmic apparatus includes a base 2, a face supporting unit 4 attached to the base 2, a movable base 6 movably provided on the base 2, and a measurement part 8. The measurement part 8 is movably provided on the movable base 6, and houses an optical system which will be described later. The movable base 6 moves on the base 2 in a horizontal direction (X direction) and a front-rear direction (Z direction) with operation of a joystick 12. The measurement part 8 is moved in a vertical direction (Y direction) by a drive mechanism 17 such as a motor when a rotation knob 12a is operated in a rotation manner. The movable base 6 includes a monitor 70 and an operation part 90. The monitor 70 displays thereon various types of information including an observation image of an examinee's eye E and a measurement result. The operation part 90 has a switch used to set various settings. In the first ophthalmic apparatus according to the first embodiment described below, the measurement part 8 includes an optical interference ocular axial length measuring optical system. In an ophthalmic apparatus according to a second embodiment which will be described later, on the other hand, a measurement part 8 includes an eye refractive power measuring optical system.

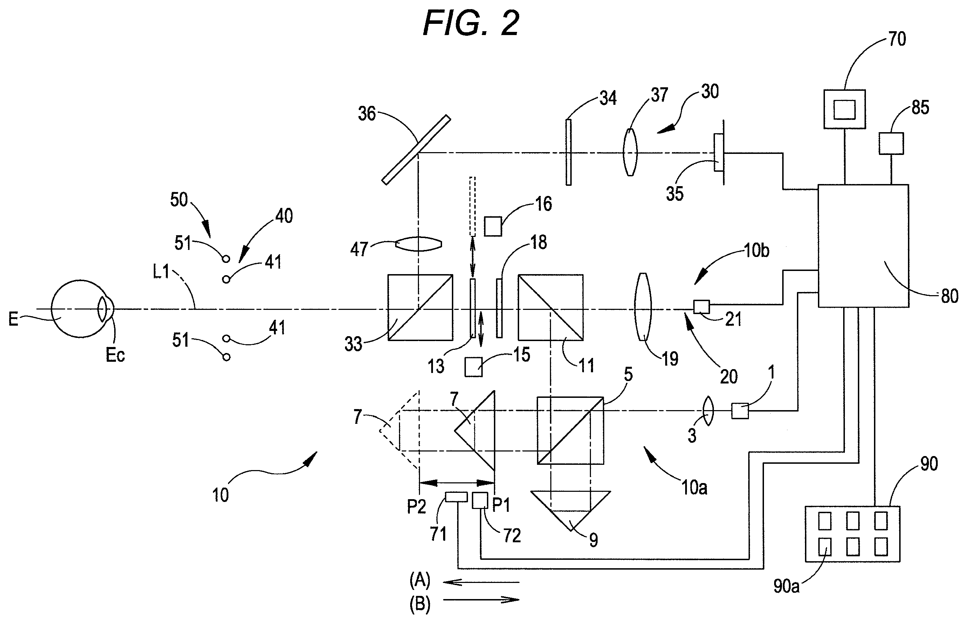

Fig. 2 is a schematic diagram illustrating the configuration of the optical system stored in the measurement part 8 of the ophthalmic apparatus. This optical system is broadly divided into an ocular axial length measuring optical system (measurement unit) 10, a kerato-projecting optical system 50, an alignment projecting optical system 40, and an anterior segment front imaging optical system 30. The kerato-projecting optical system 50 projects a target for a corneal shape measurement onto a cornea of the eye E. The anterior segment front imaging optical system 30 captures a front image of an anterior segment. The optical system illustrated in Fig. 2 is stored inside the measurement part 8.

The kerato-projecting optical system 50 has a ring-shaped light source 51 arranged with a measurement optical axis L1 as a center. The kerato-projecting optical system 50 is used to measure a corneal shape (e.g., curvature, astigmatic axial angle) by projecting a ring target onto the cornea of the examinee's eye E. As the light source 51, for example, an LED that emits infrared light or visible light is used.

The alignment projecting optical system 40 is arranged inside the light source 51, and has a projection light source 41 that emits infrared light (e.g., λ = 970 nm). The light source 41 is used to project an alignment target onto the cornea of the examinee's eye E. The light sources 51 and 41 also serve as anterior segment illuminating light sources.

The anterior segment front imaging optical system 30 is used to capture the front image of the anterior segment of the examinee's eye E. The anterior segment front imaging optical system 30 includes a dichroic mirror 33, an objective lens 47, a total reflection mirror 36, a filter 34, an imaging lens 37, and a two-dimensional imaging device 35. The two-dimensional imaging device 35 is arranged such that an imaging surface thereof is positioned substantially conjugate with the anterior segment of the examinee's eye E. The anterior segment front imaging optical system 30 captures a retro-illumination image (an image of an inner pupil) that is obtained by illuminating the pupil with fundus reflection light. The dichroic mirror 33 transmits most of the measurement light therethrough, while reflecting some of the measurement light. The dichroic mirror 33 reflects light from the light sources 51 and 41.

Anterior segment reflection light, which is obtained when the light from the projecting optical systems 40 and 50 is reflected by the anterior segment, is formed into an image on the two-dimensional imaging device 35 via the dichroic mirror 33, the objective lens 47, the total reflection mirror 36, the filter 34, and the imaging lens 37.

The ocular axial length measuring optical system 10 has a light projecting optical system 10a and a light receiving optical system 10b. The ocular axial length measuring optical system 10 projects the measurement light onto the examinee's eye E, and detects interference light that is obtained from the light reflected by the examinee's eye E. The light projecting optical system 10a includes a measurement light source 1, a collimator lens 3, a beam splitter 5, a first triangular prism (corner cube) 7, a second triangular prism 9, a polarizing beam splitter 11, a quarter wavelength plate 18, and a diffuser plate 13. The measurement light source 1 emits low coherent light (light source 1 also serves as a fixation lamp in this embodiment). The collimator lens 3 makes a light flux emitted from the measurement light source 1 into parallel light fluxes. The beam splitter 5 splits the light emitted from the light source 1. The first triangular prism 7 is arranged in a transmission direction of the beam splitter 5. The second triangular prism 9 is arranged in a reflection direction of the beam splitter 5. The diffuser plate 13 is an optical member that diffuses light in a random manner.

The diffuser plate 13 is arranged in an optical path of the light projecting optical system 10a, and serves as a speckle suppressing means to optically suppress a speckle noise generated on the retro-illumination image. In this embodiment, the diffuser plate 13 is arranged in a position out of an optical path of the imaging optical system 30 and in a common optical path for a first measurement light and a second measurement light. That is, when the retro-illumination image is captured, the diffuser plate 13 is moved into the optical path and vibrated in a vertical direction relative to an optical axis L1 so that the speckle noise is removed from the retro-illumination image. In this embodiment, the diffuser plate 13 is vibrated in the vertical direction relative to the optical axis L1. However, the diffuser plate 13 may be rotated in the optical path.

A first drive part 16 includes a drive source such as a motor, and is arranged to make the diffuser plate 13 move into or out of the optical path. A second drive part 15 includes a drive source such as a motor, and is arranged to allow the diffuser plate 13 moved into the optical path to be vibrated in a vertical direction relative to the optical axis. The light receiving optical system 10b is arranged to receive light (interference light) obtained by interference of cornea reflection light obtained by reflection of the measurement light by the cornea and fundus reflection light obtained by reflection of the measurement light by the fundus. The light receiving optical system 10b includes the dichroic mirror 33, the quarter wavelength plate 18, the polarizing beam splitter 11, a condenser lens 19, and a light receiving device 21.

The light (linear polarized light) emitted from the light source 1 is collimated by the collimator lens 3, and is then split by the beam splitter 5 into the first measurement light and the second measurement light. The first measurement light is reflected back by the triangular prism 7, while the second measurement light is reflected by the triangular prism 9 and deflected back. Subsequently, the beam splitter 5 combines the first measurement light and the second measurement light. The combined light is reflected by the polarizing beam splitter 11, and then is converted into circular polarized light by the quarter wavelength plate 18. The circular polarized light irradiates at least the cornea and the fundus of the examinee's eye E via the diffuser plate 13, which vibrates in the vertical direction relative to the optical axis, and the dichroic mirror 33. At this time, when the measurement light flux including the circular polarized light is reflected by the cornea and the fundus of the examinee's eye E, a phase of the light flux is shifted by a 1/2 wavelength.

The cornea reflection light and the fundus reflection light pass through the dichroic mirror 33 and the diffuser plate 13 (part of the fundus reflection light is reflected by the dichroic mirror 33). The both refection lights having passed through the diffuser plate 13 are converted into linear polarized light by the quarter wavelength plate 18. After passing through the polarizing beam splitter 11, the both reflection lights are condensed by the condenser lens 19 and then received as the interference light (obtained by interference of one portion of light reflected by the fundus and another portion of light) by the light receiving device 21. The light receiving device 21 outputs an interference signal based on the interference light received.

The triangular prism 7 is used as an optical-path-length changing member to change an optical path length. The triangular prism 7 is linearly moved in the optical axis direction by the driving of a drive part 71 (e.g., motor) relative to the beam splitter 5. The optical-path-length changing member may be a triangular mirror. The position of the prism 7 during driving is detected by a position detecting sensor 72 (e.g., a potentiometer, an encoder).

In the above configuration, the cornea reflection light and the fundus reflection light are made to interfere. However, the configuration is not limited thereto. Alternatively, the first ophthalmic apparatus may include an optical interference optical system including a sample arm, a reference arm, a beam splitter (light splitting member) for splitting light emitted from a light source, and a light receiving device for receiving interference light. In such an optical interference optical system, the light receiving device receives the interference light obtained by interference of measurement light with which an examinee's eye is irradiated via the sample arm and reference light from the reference arm. In this case, the optical-path-length changing member is arranged on at least any of the sample arm and the reference arm.

In the above configuration, although the linear movement of the prism 7 changes the optical path length of the reference light, this configuration is not necessarily required. For example, the first ophthalmic apparatus may be configured to allow the optical path length of the reference light to be changed by an optical delay mechanism via a rotating reflector (see, for example, JP-A-2005-160694).

In the first ophthalmic apparatus, the measurement light source 1 of the measuring optical system 10 is used as a light source for capturing a retro-illumination image. The light from the measurement light source 1 as the light source for capturing a retro-illumination image is projected onto the fundus of the examinee's eye E through an optical path that is substantially the same as that of the light projecting optical system 10a. Then, an inner pupil of the examinee's eye E is illuminated from behind with the reflected light from the fundus. This reflected light is emitted from the pupil of the examinee's eye E and formed into an image by the two-dimensional imaging device 35 through a path that is substantially the same as that of the anterior segment reflection light described above. Therefore, the retro-illumination image of the inner pupil of the examinee's eye E is obtained.

Next, a control system is described. A control part 80 controls the apparatus as a whole and calculates a measurement result. The control part 80 is connected to the light sources 1, 51, and 41, the drive mechanism 17, the first drive part 16, the second drive part 15, the light receiving device 21, the imaging device 35, the monitor 70, a memory 85, and the like. The control part 80 is also connected to an operation part 90 for various input operations. The memory 85 stores therein a software program for calculation of an ocular axial length by the control part 80 in addition to various control programs.

The operation part 90 includes a mode switching switch 90a (mode switching means). The mode switching switch 90a outputs a switching signal. For example, the switching signal serves as a signal for switching between a first mode (first measurement mode) in which a measuring optical system measures an ocular axial length by receiving interference light and a second mode (second measurement mode) in which an imaging optical system captures a retro-illumination image picture that is an inner pupil image. The operation part 90 may be a general-purpose interface such as a mouse as an operation input part, or a touch panel. The retro-illumination image picture is, for example, an image including a retro-illumination image and an iris portion in a periphery thereof. The terms "retro-illumination image" and "retro-illumination image picture" may be used interchangeably throughout the description of the embodiments.

The control part 80 switches modes of the first ophthalmic apparatus between the first mode and the second mode based on the switching signal from the mode switching switch 90a. The control part 80 stops a movement of the diffuser plate 13 (speckle suppressing means) in the first mode, whereas the control part 80 allows the movement of the diffuser plate 13 in the second mode.

More specifically, the control part 80 controls the driving of the first drive part 16 and the second drive part 15, thereby controlling the movement of the diffuser plate 13 into and out of the optical path and controlling the vibration of the diffuser plate 13. When the ocular axial length is measured, the diffuser plate 13 is moved out of the optical path and the vibration thereof is stopped.

In the first mode, the diffuser plate 13 is moved out of the optical path, and the operations of the first drive part 16 and the second drive part 15 are stopped. When the first ophthalmic apparatus is switched from the first mode to the second mode, the control part 80 operates the first drive part 16 to move the diffuser plate 13 into the optical path. The control part 80 also operates the second drive part 15 to vibrate the diffuser plate 13. When the first ophthalmic apparatus is switched from the second mode to the first mode by operation of the mode switching switch 90a, on the other hand, the control part 80 stops the operation of the second drive part 15, thereby stopping the vibration of the diffuser plate 13. Then, the control part 80 operates the first drive part 16 to move the diffuser plate 13 out of the optical path. Once the diffuser plate 13 is moved out of the optical path, the control part 80 stops the operation of the first drive part 16.

The operation of the first ophthalmic apparatus having the above configuration will be described below. First, the description is given of a case where the first ophthalmic apparatus is set to the first mode. An examiner moves the first ophthalmic apparatus in vertical, horizontal, and front-rear directions by use of an operation means such as the joystick 12 while looking at an alignment state of the first ophthalmic apparatus (measurement optical axis) with respect to an examinee's eye E displayed on the monitor 70. The examiner places the first ophthalmic apparatus in a predetermined position with respect to the examinee's eye E. In this case, the examiner requests the examinee to fixate a fixation target.

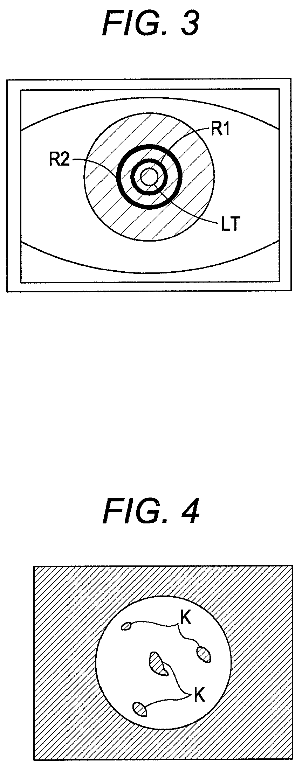

Fig. 3 is a diagram illustrating an anterior segment observation screen displaying an anterior segment image of the examinee's eye E captured by the imaging device 35. During alignment (adjusting a position of a measurement optical axis of the apparatus to a proper position), the light sources 51 and 41 are turned on. The examiner performs alignment of the first ophthalmic apparatus in the vertical and horizontal directions such that a reticle LT and a ring target Rl by the light source 41 are formed to be concentric as shown in Fig. 3. In this embodiment, the reticle LT represents an alignment reference position that is displayed electronically, the alignment reference position coinciding with a corneal apex position and being set as a position of the optical axis L1 of the first ophthalmic apparatus. The examiner also performs alignment of the first ophthalmic apparatus in the front-rear direction such that the ring target R1 is in focus. The ring target R1 has an outer side on which a ring target R2 is displayed by the light source 51.

### <Calculation of Ocular Axial length >

After completion of the alignment, a trigger signal for initiation of measurement is output automatically or manually. When the measurement light source 1 is turned on by the control part 80, the examinee's eye E is irradiated with measurement light by the ocular axial length measuring optical system 10. The reflected light obtained by reflection of the measurement light by the examinee's eye E is incident on the light receiving device 21 of the light receiving optical system 10b.

The control part 80 controls the drive part 71 to move the first triangular prism 7 in a reciprocating manner. The control part 80 calculates an ocular axial length based on a light receiving signal (interference signal) output from the light receiving device 21 and a timing at which the interference light is detected by the light receiving device 21.

The memory 85 stores information on the ocular axial length of the examinee's eye E obtained. After completion of the predetermined number of measurements (or after the predetermined number of ocular axial length values of the examinee's eye is obtained), the control part 80 ends the reciprocating movement of the prism 7 and allows the prism 7 to return to its initial position. In the first mode, an ocular axial length, a corneal shape, and the like may be measured appropriately.

### <Capturing Retro-illumination Image>

Next, a description is given of a case where the first ophthalmic apparatus is set to the second mode. When the examiner operates (selects) the mode switching switch 90a such that the first ophthalmic apparatus is switched to the second mode, the control part 80 switches the first ophthalmic apparatus from the first mode to the second mode. Alternatively, the control part 80 may be configured to automatically switch the first ophthalmic apparatus from the first mode to the second mode. In the second mode, the control part 80 operates the first drive part 16 to move the diffuser plate 13 into the optical path. Subsequently, the control part 80 operates the second drive part 15 to vibrate the diffuser plate 13 in the optical path.

Subsequently, the control part 80 turns off the light sources 51 and 41, but turns on the light source 1. The light emitted from the light source 1 passes through the diffuser plate 13 and is reflected by the fundus. This fundus reflection light illuminates the inside of a crystalline lens of the examinee's eye E, and is then emitted from the pupil. The fundus reflection light emitted from the pupil is reflected by the dichroic mirror 33, and is then captured as a retro-illumination image by the imaging device 35. The captured retro-illumination image is displayed on the monitor 70. Accordingly, a dark cloud K corresponding to a portion having opacity caused by cataract and the like can be recognized (see Fig. 4).

Herein, a path of light emitted from a projecting system is taken into consideration; a wavelength of the light is shorter than or substantially the same as concavity and convexity on a surface such as a fundus of an examinee's eye. Thus, light fluxes irradiated toward the fundus of the examinee's eye E may be superposed each other on the fundus with complicated phase changes, causing the possibility of generating the interference. In such a case, if a retro-illumination image is captured based on the fundus reflection light, uneven spots (speckle noise) are generated on a retro-illumination image picture.

Such uneven spots on the retro-illumination image picture can be removed by providing the diffuser plate 13 in the optical path. That is, the vibration of the diffuser plate 13 in a vertical direction relative to an optical axis in the optical path generates a phase difference to the measurement light traveling toward the fundus, so that an interference state of the measurement light on the fundus is changed. Consequently, patterns of the speckle noise on the retro-illumination image picture change on a time bases, and thus the retro-illumination image picture is integrated within a response time of the optical system. Accordingly, the patterns of the speckle noise are averaged. In this case, for example, the diffuser plate 13 is preferably vibrated at an adequate speed such that the patterns of the speckle noise change repeatedly within a time in which the imaging device 35 obtains one frame picture.

When an imaging switch 12b provided on the joystick 12 is operated, the retro-illumination image picture is obtained and stored in the memory 85. The retro-illumination image picture can be output on the monitor 70 or to a printer, for example. In this embodiment, the image is obtained by operation of the imaging switch 12b. However, the first ophthalmic apparatus may be configured to automatically capture the image.

According to the first ophthalmic apparatus, therefore, when an image of an inner pupil such as a retro-illumination image picture is captured, uneven spots over the image can be removed. Thus, an examiner can identify opacity caused by cataract without misidentifying the uneven spots as the opacity.

In this embodiment, the diffuser plate 13 is moved when the retro-illumination image is captured. Such movements of the diffuser plate 13 allow speckle noise to be removed from the retro-illumination image, thereby obtaining a good retro-illumination image that is suitable for checking a progression state of cataract. When an ocular axial length is measured, on the other hand, the movements of the diffuser plate 13 stop, thereby ensuring optical interference between fundus reflection light and other light. Thus, the ocular axial length can be accurately measured.

In this embodiment, the diffuser plate 13 is used as an optical member for removing the speckle noise. Alternatively, for example, a prism may be used as an optical member for removing the speckle noise. When the prism is used, rotation movement of the prism by a drive part rotates a light flux eccentrically. This leads to a change of a scattering state of the light flux on a fundus, so that the speckle noise can be removed. Moreover, a member such as a crystal depolarization plate, a bundle fiber, a photonic crystal, and a liquid crystal plate may be used to reduce coherence. This also can reduce the speckle noise.

In this embodiment, the diffuser plate 13 is arranged to be moved into between the polarizing beam splitter 11 and the dichroic mirror 33, but is not limited thereto. The diffuser plate 13 may be moved into any position as long as being out of the optical path of the imaging optical system 30.

Moreover, the position of the diffuser plate 13 to be moved into is preferably in a common optical path for the first measurement light and the second measurement light (e.g., between the light source 1 and the beam splitter 5). This provides an effect of the optical member (diffuser plate 13) for removing the speckle noise on the entire measurement light flux. In the case where the diffuser plate 13 is provided in one of split optical paths, light in another optical path is not changed.

When the prism is used, the light flux is preferably moved on the fundus. Thus, the prism is preferably arranged in a position that is out of the optical path of the imaging optical system 30 and is out of conjugation with the fundus.

In this embodiment, the measurement light source 1 includes an SLD serving as a light source that emits low coherent light, but is not limited thereto. The measurement light source 1 may be any light source that emits light having coherence. For example, the measurement light source 1 may be a multimode laser diode serving as a high coherent light source.

As described above, for example, the prism serving as a light flux deflecting member can be used as the speckle removing means of the first ophthalmic apparatus. In this case, the light flux deflecting member may be arranged in a position substantially conjugate with the pupil. In this manner, even if the light flux deflecting member is moved during measurement of an ocular axial length, a movement of cornea reflection light can be reduced. In addition, reduction of coherence may be suppressed by a certain amount.

### <Second Embodiment>

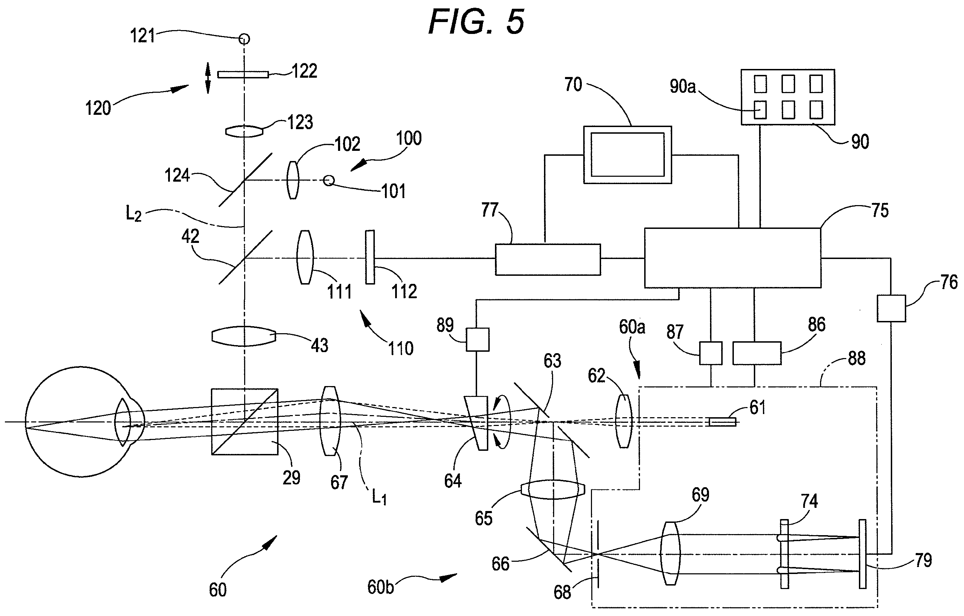

A second embodiment will be described below based on the accompanying drawings. Fig. 5 is a schematic diagram illustrating the configuration of an optical system and a control system in an ophthalmic apparatus (a second ophthalmic apparatus; eye refractive power measuring apparatuses) according to a second embodiment.

The optical system is broadly divided into a measuring optical system 60, an observing optical system 110, an alignment projecting optical system 100, and a fixation target optical system 120.

The measuring optical system 60 includes a projecting optical system 60a and a light receiving optical system 60b. The projecting optical system 60a projects a light flux having a spot shape onto a fundus via a center portion of a pupil of an examinee's eye. The light receiving optical system 60b extracts the light reflected by the fundus in a ring shape from a periphery of the pupil.

The projecting optical system 60a includes an infrared point light source 61 such as an SLD, a relay lens 62, a hole mirror 63, a prism 64 rotationally driven around an optical axis L by a drive part 89, and a measurement objective lens 67. These members 61 through 64 and 67 are arranged on a measurement optical axis L1 and arranged in sequence so as to face the examinee's eye. The light source 61 is arranged in a position having a substantially conjugate relationship with the fundus of the examinee's eye. The hole mirror 63 has a hole that is arranged in a position having a conjugate relationship with the pupil. The prism 64 is arranged away from a position that has a conjugate relationship with the pupil and fundus of the examinee's eye. The prism 64 deflects the light flux passing therethrough with respect to the optical axis L1. A beam splitter 29 serving as an optical path splitting member is arranged between the measurement objective lens 67 and the examinee's eye. The beam splitter 29 reflects the reflected light from an anterior segment of the examinee's eye toward the observing optical system 110. The beam splitter 29 guides the light flux from the fixation target optical system 120 to the examinee's eye. Part of the light emitted from the infrared point light source 61 is reflected by the fundus as the fundus reflection light, and is then reflected by the beam splitter 29, thereby being guided to the observing optical system 110. On the other hand, the beam splitter 29 transmits therethrough another part of the fundus reflection light, thereby guiding to the light receiving optical system 60b. Among the fundus reflection light originally from the infrared point light source 61, the fundus reflection light guided to the observing optical system 110 is used to capture a retro-illumination image picture (described later).

The light receiving optical system 60b shares the measurement objective lens 67, the prism 64, and the hole mirror 63 with the projecting optical system 60a. The light receiving optical system 60b includes a relay lens 65, a mirror 66, a light receiving aperture 68, a collimator lens 69, a ring lens 74, and an imaging device 79. The relay lens 65 is arranged in an optical path in a reflecting direction of the hole mirror 63. The relay lens 65 is arranged in an optical path in a reflecting direction of the mirror 66. The imaging device 79 serves as a two-dimensional light receiving device such as a CCD.

The light receiving aperture 68 and the imaging device 79 are arranged in positions that have conjugate relationships with the fundus of the examinee's eye. An output from the imaging device 79 is input to a control part 75 via an image processing part 76.

The light source 61 of the projecting optical system 60a, the light receiving aperture 68 of the light receiving optical system 60b, the collimator lens 69, the ring lens 74, and the imaging device 79 are integrally movable as a movable unit 88 in an optical axis direction. The drive part 86 moves the movable unit 88 in the optical axis direction according to a spherical refractive error (spherical refractive power) of the examinee's eye. That is, the drive part 86 corrects the spherical refractive error by arranging the light source 61, the light receiving aperture 68, and the imaging device 79 in positions that are optically conjugate with respect to the fundus of the examinee's eye. A movement position of the movable unit 88 is detected by a potentiometer 87. The hole mirror 63 and the ring lens 74 are arranged in positions that have conjugate relationship with the pupil of the examinee's eye with a certain magnification regardless of a movement amount of the movable unit 88.

An optical axis L2 is coaxially provided with the optical axis L1 by the beam splitter 29. The optical axis L2 has thereon an observation objective lens 43, a half mirror 42, a dichroic mirror 124, a light projecting lens 123, a fixation target 122, and a visible light source 121 arranged in sequence. These members from the observation objective lens 43 to the light source 121 form the fixation target optical system 120. The light source 121 and the fixation target 122 move in an optical axis L2 direction, thereby fogging the examinee's eye. The light source 121 illuminates the fixation target 122. A light flux from the fixation target 122 travels through the light projecting lens 123, the dichroic mirror 124, the half mirror 42, and the objective lens 43, and is reflected by the beam splitter 29 to travel toward the examinee's eye. The examinee's eye fixates the fixation target 122.

The alignment projecting optical system 100 projects an alignment target image from a front of the examinee's eye. Near infrared rays from a light source 101 are condensed by a condenser lens 102 and are formed into a substantially parallel light flux via the dichroic mirror 124, the half mirror 42, and the objective lens 43. The parallel light flux is reflected by the beam splitter 29 and projected onto the examinee's eye.

The observing optical system 110 includes an imaging lens 111 and an imaging device 112 arranged on a side that undergoes the reflection of the half mirror 42. An output of the imaging device 112 is input to the monitor 70 via an image processing part 77. The anterior segment of the examinee's eye is formed into an image on an imaging device surface of the imaging device 112 via the beam splitter 29, the objective lens 43, the half mirror 42, and the imaging lens 111. The observation image obtained by image formation is displayed on the monitor 70. The observing optical system 110 may also serve as an optical system that detects an alignment target image formed on a cornea of an examinee's eye and as an optical system that detects a pupil position. In such a case, the image processing part 77 detects a target image position and the pupil position.

The control part 75 analyzes ring images obtained via the image processing part 76, and calculates an eye refractive power of the examinee's eye. The control part 75 also controls the second ophthalmic apparatus as a whole. The control part 75 is connected with an operation part 90 including a mode switching switch 90a. For measurement of the same eye, the mode switching switch 90a switches between a first mode in which the eye refractive power is measured and a second mode in which a retro-illumination image picture as an image of an inner pupil is captured.

### <Measurement of Eye Refractive Power>

The operation of the second ophthalmic apparatus having the above configuration will be described below. When the second ophthalmic apparatus is set to the first mode by the mode switching switch 90a, the control part 75 turns on the light source 61 and controls the drive part 89 to rotate the prism 64 at a high speed. Subsequently, a position of the measurement optical axis L1 is adjusted to a center of a pupil of an examinee's eye (or center of cornea), and the control part 75 allows the light source 61 to emit infrared light when a trigger signal for initiation of measurement is output.

The infrared light output from the light source 61 passes through the relay lens 62, the hole mirror 63, the prism 64, the objective lens 67, and the beam splitter 29, and is formed into a point light source image having a spot shape on the fundus of the examinee's eye. Herein, the rotation of the prism 64 around the optical axis eccentrically rotates a projected pupil image (projected light flux on the pupil) of the hole of the hole mirror 63 at a high speed.

The point light source image projected onto the fundus is reflected/scattered at the fundus and is emitted from the examinee's eye. Part of this fundus reflection light is reflected by the beam splitter 29, whereas another part passes through the beam splitter 29. The fundus reflection light having passed through the beam splitter 29 is condensed by the objective lens 67, then passes through the prism 64 rotating at a high speed, the hole mirror 63, the relay lens 65, and the mirror 66, and is condensed again in a position of the light receiving aperture 68. Thereafter, the condensed fundus reflection light is formed into an image having a ring shape on the imaging device 79 by the collimator lens 69 and the ring lens 74. Herein, the reflected light flux from the fundus passes through the prism 64 that is substantially the same as the projecting optical system 60a. In any optical system beyond the prism 64, therefore, the reflected light flux is treated as if there was no eccentricity of the projected light flux/reflected light flux (received light flux) on the pupil.

An output signal from the imaging device 79 is detected and processed by the image processing part 76. When an examinee's eye is an emmetropic eye, a position of the imaging device 79 and a position of the fundus have a conjugate relationship. This allows the fundus reflection light to be incident as a parallel light flux on the ring lens 74. Therefore, a ring image is formed on the imaging device 79, the ring image having substantially the same size as the ring lens 74. When an examinee's eye has a refractive error (spherical refractive error) in a spherical refractive component, on the other hand, a ring image to be formed on the imaging device 79 has a radius that increases in proportion to an amount of the spherical refractive error. When an examinee's eye has an astigmatic refractive error, a ring image to be formed on the imaging device 79 has an oval shape according to the astigmatic refractive error. Consequently, shape analysis of the ring image formed on the imaging device 79 can determine refractive errors in respective meridian directions. These refractive errors undergo a predetermined process, so that refractive value S (spherical dioptic power), value C (astigmatic power), and value A (astigmatic axial angle) of the examinee's eye can be determined. A size of the ring image can be determined as a center position of an edge of the ring image, a barycentric position of a light quantity level, or a peak position of the light quantity level.

In an actual measurement, the fixation target 122 is once placed in a position conjugate with the fundus based on a refractive power obtained from a preliminary measurement. Subsequently, the fixation target 122 is moved such that fog is applied with appropriate diopter. The actual measurement is executed in a state in that the examinee's eye is being applied with fog. A target light flux of the fixation target optical system 120 travels toward the examinee's eye via the beam splitter 29 arranged on a side closer to the examinee's eye relative to the prism 64. Thus, the examinee's eye can gaze at a fixation target in a stable manner.

### <Capturing Retro-illumination Image>

When the mode switching switch 90a outputs a switching signal for switching to a second mode (may be automatically switched), the control part 75 turns off the light source 101, but turns on the light source 61. The light emitted from the light source 61 passes through the prism 64 and is reflected by the fundus. This fundus reflection light illuminates the inside of a crystalline lens of the examinee's eye, and is then emitted from the pupil. Part of the fundus reflection light emitted from the pupil is reflected by the dichroic mirror 29, passes through the imaging lens 111, and is formed as a retro-illumination image on an imaging device surface of the imaging device 112. The formed retro-illumination image is displayed on the monitor 70.

In the second ophthalmic apparatus, an SLD serving as a light source having coherence (high interference) is used as the measurement light source 61 of the measuring optical system 60. When the retro-illumination image is captured based on the fundus reflection light provided by the measurement light source 61, uneven spots (speckle noise) are generated on the retro-illumination image picture as similar to the first embodiment.

In the second embodiment, the prism 64 serving as a light deflection means is used as a speckle suppressing means. That is, the prism 64 is rotated, so that the measurement light traveling toward the fundus from the measurement light source 61 is deflected with respect to the fundus. This prism 64 is arranged in a position that is not conjugate with the fundus, thereby removing the speckle noise from the retro-illumination image. In other words, the rotation of the prism 64 arranged in the position being not conjugate with the fundus eccentrically rotates the spot-shaped light flux (point light source image) projected on the fundus of the examinee's eye at a high speed. Therefore, the measurement light flux is moved on the fundus, leading to a change of a scattering state of the light flux on the fundus. As a result, patterns of the speckle noise on the retro-illumination image vary during a time in which the imaging device 112 accumulates the light fluxes. Thus, the patterns of the speckle noise are averaged (neutralization), so that influences of the speckle noise on the retro-illumination image can be removed.

In the second ophthalmic apparatus, the prism 64 is arranged between the hole mirror 63 and the objective lens 67, but not limited thereto. The prism 64 only needs to be arranged in a position that is out of conjugation with the fundus and is out of the optical path of the observing optical system 110. For example, the prism 64 may be arranged between the objective lens 67 and the beam splitter 29. The prism 64 may also be arranged in a position that is not only out of the conjugation with the fundus, but also out of the conjugation with the pupil. Therefore, the speckle noise can be removed, and the measurement light flux can be deflected on the pupil, thereby measuring an average refractive power of the eye.

According to the second ophthalmic apparatus, therefore, when an image of an inner pupil such as a retro-illumination image picture is captured, uneven spots across the image can be removed. Thus, an examiner can identify opacity caused by cataract without misidentifying the uneven spots as the opacity.

In the first and second embodiments described above, when a retro-illumination image is captured, an image of an inner pupil is preferably captured by the imaging device by controlling the drive mechanism 17 (drive means) in a state that the measurement part 8 is moved relative to an examinee's eye such that a corneal apex of the eye and an imaging optical axis of the imaging optical system are displaced. That is, when the ophthalmic apparatus is set to the second mode based on the switching signal from the mode switching switch 90a, the control part controls the drive means such that the corneal apex of the examinee's eye and the imaging optical axis of the imaging optical system are displaced. Subsequently, the retro-illumination image is captured by the imaging device.

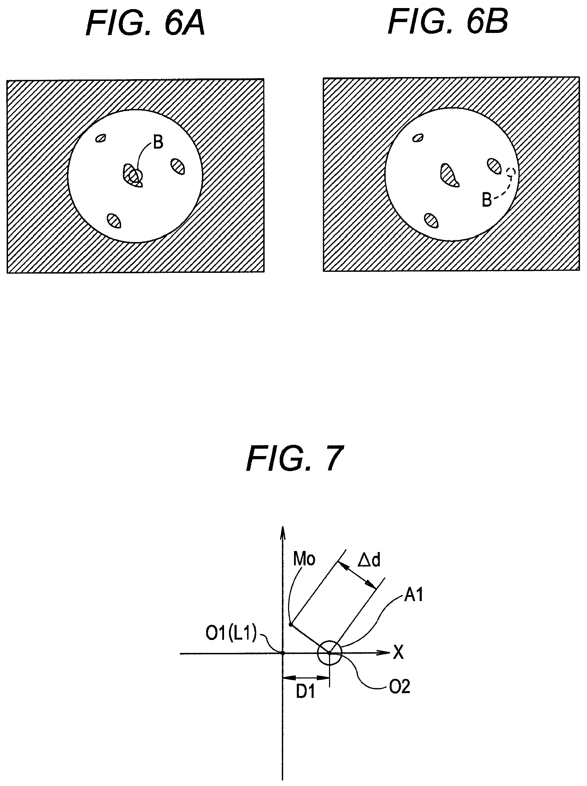

Generally, an ocular axial length and an eye refractive power are measured in a state that a cornea apex and an imaging optical axis coincide with each other. However, when a retro-illumination image is to be captured in such a state, a center portion of a retro-illumination image picture has a corneal luminescent spot B reflected by a measurement light source, causing not only an increase in difficulty of observing the retro-illumination image picture by an examiner, but also an increase in the likelihood of misidentifying an opacity portion and the corneal luminescent spot (see Fig. 6A). The displacement of the corneal apex and the imaging optical axis can reduce the luminance of the corneal luminescent spot B, thereby facilitating the observation of the retro-illumination image by the examiner while reducing the likelihood of misidentifying the opacity portion and the corneal luminescent spot (see Fig. 6B). Such a technique for displacing the corneal apex and the imaging optical axis by use of the drive mechanism 17 is applied to a configuration including a light source having high coherence, but not limited thereto. This technique may be applied to a configuration including an LED having low coherence.

Now, the technique for displacing the corneal apex and the imaging optical axis using the drive mechanism 17 will be described in detail with reference to Figs. 6A and 6B. In the description below, the first ophthalmic apparatus is used as an example. When the second mode is selected by operation of the mode switching switch 90a, the control part 80 switches the first ophthalmic apparatus to the second mode from the first mode.

After the retro-illumination image picture is displayed on the monitor 70, the control part 80 detects an XY coordinate position in which a luminance value becomes a peak value as a corneal apex position (corneal luminescent spot position) Mo based on the retro-illumination image captured by the imaging device 35. The control part 80 determines a displacement amount Δd between a luminescent spot target position 02 (D1, 0) in XY directions and the corneal apex position Mo (see Fig. 7), the luminescent spot target position 02 being determined on the imaging device 35 beforehand. The control part 80 uses the drive mechanism 17 to adjust the position of the measurement part 8, thereby adjusting the displacement amount Δd.

The luminescent spot target position 02 is used during the second mode. In the second mode, the measurement part 8 is moved such that the luminescent spot target position 02 approaches the corneal apex position Mo. The luminescent spot target position 02 represents a position that is displaced by a predetermined amount D1 with respect to an optical axis position O1 corresponding to an imaging optical axis L1 on an image surface of the imaging device 35 of the measurement part 8. In other words, the displacement amount Δd to be detected is offset by an amount of D1 with respect to the optical axis position O1. The amount D1 is set in consideration of size and/or brightness of the corneal luminescent spot B, an imaging state of the retro-illumination image, and the like.

The luminescent spot target position 02 has a periphery in which an acceptable range A1 is set. Upon completion of the position adjustments of the measurement part 8, the control part 80 automatically controls the drive mechanism 17 such that the displacement amount Δd is in the acceptable range A1.

After completion of the position adjustments of the measurement part 8, the control part 80 determines whether the position adjustments of the measurement part 8 (measurement optical axis) in the XY directions are appropriate based on whether or not the displacement amount Δd is continuously present in the acceptable range A1 for longer than a certain time period (e.g., a time needed to process 10 frame images or 0.3 seconds).

When the displacement amount Δd in XYZ directions is present in the acceptable range A1 for longer than the certain time period, the control part 80 stops the driving of the drive mechanism 17 and outputs a position adjustment completion signal. The control part 80 detects the displacement amount Δd as needed even after completion of the position adjustments. In the case where the displacement amount Δd becomes out of the acceptable range A1 before completion of imaging of the retro-illumination image, the control part 80 resumes the automatic position adjustment (automatic alignment) of the measurement part 8 (measurement optical axis) by the drive mechanism 17. That is, the control part 80 controls the measurement part 8 to track the examinee's eye such that the displacement amount Δd satisfies the acceptable range A1.

Consequently, the corneal luminescent spot can be moved from the retro-illumination image picture displayed on the monitor 70. Thus, the retro-illumination image picture can be observed without an influence of the corneal luminescent spot. The position adjustment described above may be executed by use of an anterior segment observation image and the corneal luminescent spot at a stage prior to turning off the light sources 41 and 51.

The corneal luminescent spot may be moved by adjusting the position of the measurement part 8 while the position coordinates of the retro-illumination image are being compared with those of the corneal luminescent spot. Specifically, the control part 80 can detect the position of the corneal luminescent spot by detection of the position in which the luminance value becomes the peak value in the retro-illumination image captured by the imaging device 35.

Next, the control part 80 detects position coordinates of an outer circumference of the retro-illumination image by detection of an edge of the retro-illumination image. The control part 80 compares the position coordinates of the corneal luminescent spot with those of the outer circumference of the retro-illumination image. The control part 80 moves the measurement part 8 such that the position coordinates of the corneal luminescent spot are substantially the same as those of the outer circumference of the retro-illumination image or are outside thereof. Alternatively, the control part 80 may move the corneal luminescent spot to a position that is an inner side relative to the outer circumference of the retro-illumination image as long as the position leads to reduction of the luminance of the corneal luminescent spot B on the retro-illumination image (the position in which the corneal luminescent spot B is difficult to identify).

The control part 80 may not need to detect the position of the corneal luminescent spot unlike the above description. That is, the control part 80 may stop the movement of the measurement part 8 when the size/brightness of the corneal luminescent spot reaches a predetermined acceptable range. The control part 80 may not need to detect the corneal luminescent spot. That is, the control part 80 may move the measurement part 8 by a predetermined amount by control of the driving of the drive mechanism 17 based on the switching signal for switching to the second mode, the switching signal being output after completion of the first mode.

### <Improvement of Measurable Rate>

The above configuration can avoid an occurrence of generating uneven spots across an image when the image of an inner pupil such as a retro-illumination image picture is captured. Accordingly, an examiner can readily observe opacity caused by cataract, thereby improving a measurable rate of a measurement (success rate of measurement) of an examinee's eye.

For example, when there is an opacity portion in a corneal apex and a measurement optical axis of the ophthalmic apparatus is in a position in which the corneal apex passes through, the measurement light is scattered by the opacity. This may cause the measurement to fail. In such a case, the control part 80 changes a position of the measurement optical axis on the pupil, so that the measurement light can avoid passing through the opacity portion. As a result, the scattering of the measurement light is suppressed, thereby improving the measurable rate.

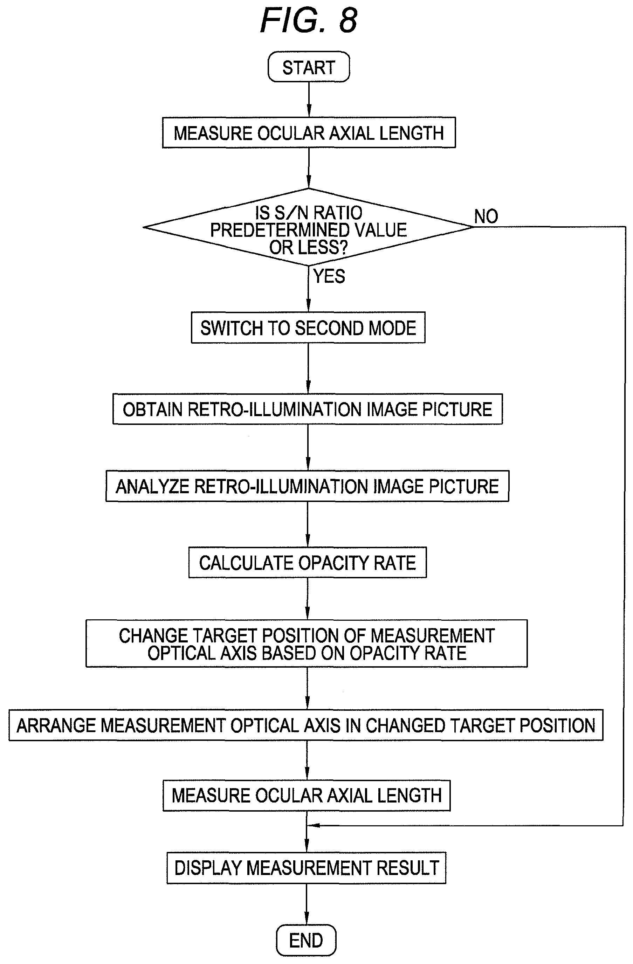

Referring to a flowchart of Fig. 8, an exemplary procedure for improving the measurable rate of measuring an examinee's eye is described. In the following description, the first ophthalmic apparatus, including the measurement part 8 having an optical interference ocular axial length measuring optical system, is used as an example.

First, an ocular axial length is measured. When a measurement optical axis is positioned on a corneal center, a trigger signal for initiation of measurement is automatically or manually output. The control part 80 calculates the ocular axial length based on the trigger signal. The control part 80 switches modes of the first ophthalmic apparatus from a first mode to a second mode based on the measurement result of the ocular axial length obtained.

For example, when an S/N ratio of interference signal for measurement of the ocular axial length obtained is lower than or equal to a predetermined value, the control part 80 switches the mode. Alternatively, the control part 80 may allow the monitor 70 to display accordingly. In such a case, the examiner selects whether or not to switch the mode.

After switching the mode, the control part 80 captures the retro-illumination image using the imaging device 35. The control part 80 allows the monitor 70 to display the retro-illumination image captured (see to Fig. 4). When the trigger signal for initiation of imaging is automatically or manually output, the control part 80 obtains a retro-illumination image (retro-illumination image picture) for the purpose of analysis. The control part 80 analyzes the obtained retro-illumination image picture to change a position of the measurement optical axis.

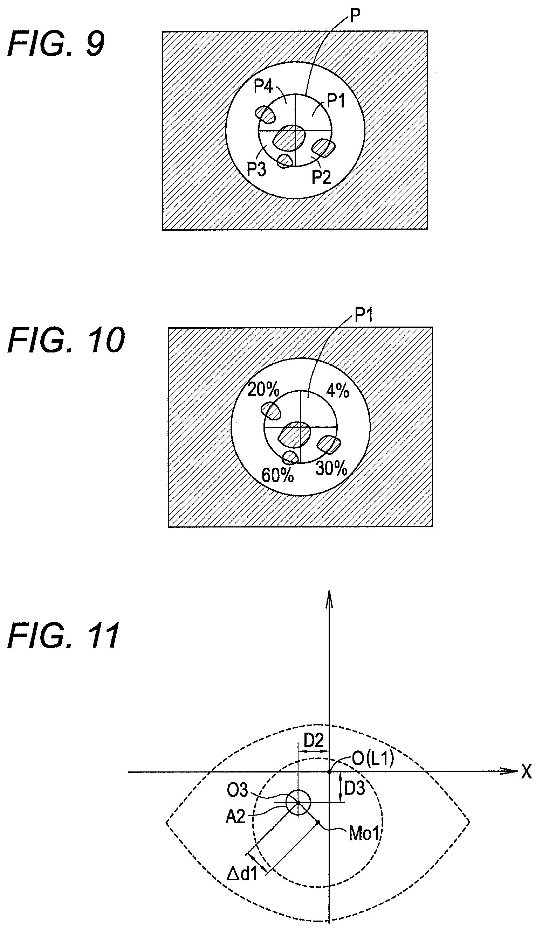

For example, Fig. 9 illustrates partitioned regions P1 through P4 within a measurable region P of the retro-illumination image picture. The control part 80 changes a position of the measurement optical axis based on opacity rates in respective partitioned regions P1 through P4.

The S/N ratio of the interference signal including reflected light from the examinee's eye becomes lower as the measurement optical axis is farther from the a corneal apex position. The lower the S/N ratio, the greater the difficulties in obtaining the interference signal. Consequently, the measurement value of the ocular axial length may not be calculated.

The measurable region P is provided at a certain distance from the corneal apex position. That is, the measurable region P serves as a region in which the S/N ratio of the interference signal including the reflected light from the examinee's eye is higher than or equal to an acceptable value when the measurement light is incident in a state that there is no opacity (e.g., region having a diameter of 1.0 mm with corneal apex as a center).

For example, an S/N ratio used to obtain a measurement value of an ocular axial length is obtained when an area of a pupil in which the measurable region P and a measurement light flux (measurement light has a diameter of 1.5 mm) overlap each other is greater than or equal to a predetermined value. An interference signal originated from light in the vicinity of a measurement optical axis L1 (center portion of measurement light flux) can provide the highest S/N ratio. Accordingly, when the vicinity of the optical axis L1 passes the measurable region P, the measurement success rate is increased.

The measurable region P is set beforehand by an experiment, a simulation, and the like. For example, a person who sets the measurable region P determines a region in which an interference signal having an S/N ratio higher than or equal to a predetermined value is obtained while moving an optical axis L1 from a corneal apex (e.g., determining a region provided at ΔD distance from the corneal apex). The measurable region P is partitioned into the partitioned regions P1 through P4 each of which is partitioned by a predetermined region. The control part 80 changes the position of the optical axis L1 to any of the partitioned regions P1 through P4 (described in detail later). An opacity rate in each partitioned region indicates a percentage of the opacity portion in proportion to the entire partitioned region (see Fig. 10).

In this embodiment, the measurable region P has a circular shape, but the shape is not limited thereto. The measurable region P may have any shape as long as an interference signal having the S/N ratio higher than or equal to the predetermined value is obtained. The control part 80 allows the retro-illumination image and a pattern indicating the measurable region P to be electronically displayed on the monitor 70. The pattern may have any shape, for example, a circle or a square.

The control part 80 obtains luminance information on the inside of the measurable region P of the retro-illumination image picture so as to calculate the opacity rate of each of the partitioned regions P1 through P4. A method for calculating the opacity rate is now described. For example, the control part 80 detects a luminance value of each pixel within the measurable region P, and determines whether or not the detected luminance value is smaller than or equal to a predetermined threshold value. Accordingly, the control part 80 determines whether each pixel belongs to an opacity portion or non-opacity portion. When the luminance value of a pixel is smaller than or equal to the predetermined threshold value, the control part 80 determines that the pixel belongs to the opacity portion. When the luminance value of a pixel is greater than the predetermined threshold value, on the other hand, the control part 80 determines that the pixel belongs to the non-opacity portion.

The control part 80 executes such determination processes with respect to all the pixels within the measurable region P. The control part 80 calculates the number of pixels belonging to the opacity portion for each of the partitioned regions P1 through P4. The control part 80 calculates the opacity rate for each of the partitioned regions. For example, an opacity rate can be expressed by numeric values from 0 (zero) to 100. An opacity rate of a partitioned region is closer to 100 as the number of pixels belonging to the opacity portion within the partitioned region is greater. On the other hand, the smaller the number of pixels belonging to the opacity portion, the closer the value to zero. Thus, an opacity rate of zero indicates no opacity portion within a partitioned region, whereas an opacity rate of 100 indicates that all the pixels within a partitioned region belong to an opacity portion.

Uneven spots generated across the retro-illumination image are removed by the diffuser plate 13, so that the control part 80 can detect opacity of cataract without misidentifying the uneven spots as the opacity.

The control part 80 changes the position of the measurement optical axis based on the calculated opacity rate. For example, opacity rates of respective partitioned regions are compared with one another by the control part 80. The control part 80 changes a position of the measurement optical axis L1 based on the comparison result such that the measurement optical axis L1 of the first ophthalmic apparatus passes the partitioned region with the lowest opacity rate.

Fig. 10 illustrates an exemplary screen on the monitor 70 displaying the retro-illumination image picture and the partitioned regions with respective calculated opacity rates.

The control part 80 allows the measurement part 8 to move in vertical and horizontal directions relative to an examinee's eye, thereby displacing the measurement optical axis L1 from the vicinity of a corneal apex. For example, the control part 80 changes the position of the measurement optical axis L1 such that a predetermined position within the partitioned region P1 having the lowest opacity rate and the measurement optical axis L1 of the first ophthalmic apparatus coincide with each other (see Fig. 11). The predetermined position is, for example, a barycentric position of the partitioned region. This predetermined position serves as a target position (target position 03 described later) of the measurement optical axis L1.

A reference position O represents a position in which the measurement optical axis L1 of the first ophthalmic apparatus coincides with the corneal apex position. As illustrated in Fig. 11, the control part 80 moves the position of the measurement optical axis to the target position 03 that is displaced from the reference position O by D2 in a left direction and by D3 in a downward direction relative to the examinee's eye. The displacement amounts (D2, D3) are calculated beforehand by an experiment, a simulation and the like such that a measurement optical axis (measurement light) passes a predetermined position of each of the partitioned regions. The calculated displacement amounts (D2, D3) are stored in the memory 85.

The control part 80 changes the position of the measurement optical axis to the target position 03. The control part 80 then turns on the light sources 51 and 41, but turns off the light source 1. The control part 80 also allows an anterior segment image to be displayed on the monitor 70.

The control part 80 executes an automatic alignment such that the measurement optical axis is arranged in the target position 03. The automatic alignment is automatic position adjustment of the measurement part 8 (measurement optical axis) by the drive mechanism 17. The control part 80 detects a corneal apex position (corneal luminescent spot position) Mo1, and determines a displacement amount Δd1 between the target position 03 and the corneal apex position Mo1 based on the detection result. The determined displacement amount Δd1 is offset by amounts of D2 and D3 with respect to the alignment reference position O.

The control part 80 controls the drive mechanism 17 such that the displacement amount Δd1 is provided within an acceptable range A2 that is set with the target position 03 as a center (operation of automatic alignment). After completion of the position alignments of the measurement part 8, the control part 80 determines whether the position adjustments of the measurement part 8 (measurement optical axis) in XYZ directions are appropriate based on whether or not the displacement amount Δd1 is continuously present within the acceptable range A2 for longer than a certain time period (e.g., a time needed to process 10 frame images or 0.3 seconds).

When the position adjustments are determined to be appropriate, the control part 80 stops the driving of the drive mechanism 17. The control part 80 detects the displacement amount Δd1 as needed even after completion of the position adjustments. In the case where the displacement amount Δd1 becomes out of the acceptable range A2, the control part 80 can resume the position adjustment. The control part 80 may control the measurement part 8 to track the examinee's eye such that the displacement amount Δd1 satisfies the acceptable range A2.

As described above, the control part 80 executes the automatic position adjustment of the measurement optical axis. After completion of the position adjustment, a trigger signal for initiation of measurement is output automatically or manually. The control part 80 calculates the ocular axial length based on the trigger signal. The control part 80 allows the measurement result of the ocular axial length to be displayed on the monitor 70, and ends the measurement.

As described above, the control part 80 changes the position of the measurement part 8, thereby moving the position of the measurement optical axis L1 of the first ophthalmic apparatus to a region having a few opacity portions.

The movement of the optical axis L1 allows more measurement light to pass through the pupil. For example, a light flux in the vicinity of a center portion of a measurement light is unlikely to be scattered by the opacity portion. This can suppress the decrease in the S/N ratio of the interference signal caused by the scattering of the light flux at the opacity portion, thereby improving the measurable rate.

In the above description, the control part 80 switches the mode when the S/N ratio of the interference signal for measurement of the ocular axial length is lower than or equal to the predetermined value. Alternatively, the control part 80 may switch the mode based on the measurement result of the ocular axial length. However, there are cases where the predetermined number of measurement results is not obtained in spite of execution of the predetermined number of ocular axial length measurements. For example, there is a case where the measurement results are obtained only twice out of five measurements. In such a case, the control part 80 may switch the mode. The control part 80 may also switch the mode when a newly obtained measurement value of the ocular axial length is out of a predetermined measurement value (abnormal value).

In the above description, the control part 80 changes the target position of the measurement optical axis based on the opacity rate. Alternatively, however, the control part 80 may determine the target position by calculating an average luminance value of each of the partitioned regions and comparing these average values, for example. In a region with a higher opacity rate, the luminance value is smaller due to the opacity, and thus the average luminance value thereof is smaller. Therefore, the control part 80 may set a partitioned region with the highest average luminance value as a target position and move the measurement optical axis to this target position. This allows the movement of the measurement optical axis to the region having a little opacity.

In the above description, the control part 80 allows the opacity rate to be displayed on the still image of the obtained retro-illumination image picture. Alternatively, the control part 80 may allow a superimposed image to be displayed on the monitor 70, the superimposed image being obtained by superimposing an analysis result of the retro-illumination image picture onto a video image of the retro-illumination image. For example, the control part 80 first executes a superimposing process of the opacity rate onto the video image of the retro-illumination image to be displayed on the monitor 70, and then detects the movement of the retro-illumination image, thereby moving the image with the opacity rate according to the movement of the retro-illumination image. The control part 80, for example, executes the superimposing process (overlaying process) by detecting a pupil portion of the retro-illumination image picture stored in the memory 85 as a reference image and pupil portions of respective video retro-illumination images successively detected, and then superimposing these images.

In the above description, the control part 80 changes the position of the measurement optical axis based on the opacity rate. Alternatively, for example, an examiner may check the opacity rate displayed on the monitor 70, and then change the position of the measurement optical axis.

For example, when changing the measurement optical axis position, the examiner uses the operation part 90 (touch panel or joystick) to select a position on the screen of the monitor 70. Herein, the position to be selected is where the measurement optical axis L1 of the first ophthalmic apparatus passes (position in which measurement light is emitted). After selection is made, the control part 80 changes the position of the measurement optical axis L1 of the first ophthalmic apparatus such that the position of the measurement optical axis L1 passes the selected position.

The examiner may set the position of the measurement optical axis L1 using an observation result of the retro-illumination image picture instead of the opacity rate displayed on the monitor 70.

In the above description, the opacity rate is displayed on the monitor 70. However, the opacity rate is not necessarily displayed on the monitor 70. For example, the control part 80 may change the position of the measurement optical axis L1 using an opacity rate retrieved from the memory 85 and the like.

In the above description, the position of the measurement optical axis L1 of the first ophthalmic apparatus is changed such that the position of the measurement optical axis L1 passes the partitioned region with the lowest opacity rate. Alternatively, for example, the control part 80 may change the position of the measurement optical axis L1 of the first ophthalmic apparatus such that the measurement optical axis L1 passes a region corresponding to an opacity rate that leads to obtaining an acceptable S/N ratio.

In the above description, the barycentric position of the partitioned region is set as the predetermined position when the position of the measurement optical axis L1 is changed. However, the predetermined position is not limited thereto. The predetermined position only needs to be set in a position to which the measurement optical axis L1 of the first ophthalmic apparatus is moved and in which the measurement optical axis L1 of the first ophthalmic apparatus is provided within the partitioned region. This predetermined position, for example, may be set in a position at a predetermined distance from the corneal apex position within the partitioned region.

When the position of the measurement optical axis L1 is changed and then the measurement result of the ocular axial length becomes out of the predetermined acceptable range, the control part 80 changes the position of the measurement optical axis L1 again and then measures the ocular axial length. The measurement optical axis L1 may be positioned displaced from a pre-change position by a predetermined value in the same partitioned region or may be in a predetermined position in a different partitioned region. When the position of the measurement optical axis L1 is changed to the position in the different partitioned region, the partitioned region to be the destination may be selected based on the opacity rate, or may be in the vicinity of a pre-change partitioned region.

When good measurement results are not obtained or measurement results are not calculated in spite of the predetermined number of changes made to the position of the measurement optical axis L1, the control part 80 may determine that an error has occurred and stop measuring the ocular axial length. Subsequently, the control part 80 may display any message of such determination and suspension on the monitor 70.

In the above description, the position of the measurement optical axis L1 is changed based on the measurement result of the obtained ocular axial length. Alternatively, however, the position of the measurement optical axis L1 may be changed after a retro-illumination image is captured.

In the above description, the control part 80 changes the position of the measurement optical axis L1 by analyzing the retro-illumination image picture obtained as the still image. Alternatively, for example, the control part 80 may capture the retro-illumination image after switching the first ophthalmic apparatus to the second mode from the first mode, and analyze the retro-illumination image displayed on the monitor 70, so that the position of the measurement optical axis L1 may be changed based on the opacity portion displayed on the retro-illumination image. The control part 80, for example, can sequentially analyze the retro-illumination images displayed on the monitor 70, thereby updating the analysis results relating to the retro-illumination images in real time.

In the above description, the control part 80 changes the position of the measurement optical axis L1, and then completes the changes of the measurement optical axis position by automatic alignment (automatic position adjustment of the measurement part 8 (measurement optical axis) by the drive mechanism 17). Alternatively, however, the examiner may manually change (adjust) the position of the measurement optical axis L1. In such a case, after the position of the measurement optical axis L1 is changed, the control part 80 changes an image to be displayed on the monitor 70 to a front image of an anterior segment. Accordingly, the examiner checks the front image of the anterior segment and adjusts the position of the measurement optical axis L1.

The control part 80 may display the retro-illumination image or the retro-illumination image picture (e.g., image including the retro-illumination image and the iris portion in a periphery thereof) on the monitor 70 by changing a display magnification thereof, so that the retro-illumination image can be more readily observed.

The foregoing detailed description has been presented for the purposes of illustration and description. Many modifications and variations are possible in light of the above teaching. It is not intended to be exhaustive or to limit the subject matter described herein to the precise form disclosed. Although the subject matter has been described in language specific to structural features and/or methodological acts, it is to be understood that the subject matter defined in the appended claims is not necessarily limited to the specific features or acts described above. Rather, the specific features and acts described above are disclosed as example forms of implementing the claims appended hereto.

## Claims

1. An ophthalmic measurement apparatus, which is an auto-refractometer or optical interference ocular axial length measuring apparatus, for measuring an examinee's eye based on an output from a light receiving device, comprising:
a measuring optical system (10, 60) for measuring the examinee's eye by projecting a measurement light flux onto a fundus of the examinee's eye and receiving the light reflected thereby, the measuring optical system (10, 60) including:
a light source (1, 61);
a light projecting optical system (10a, 60a) for projecting the light, which is at least part of the light emitted from the light source, onto a fundus of the examinee's eye; and
a light receiving optical system (10b, 60b) for guiding light including the measurement light reflected by the fundus of the examinee's eye to the light receiving device (21, 79); and
an imaging optical system (30, 110), including an imaging device (35, 112) having an imaging surface arranged in a position substantially conjugate with an anterior segment of the examinee's eye, for capturing a retro-illumination image using the imaging device by illuminating an inner pupil from behind with the measurement light reflected by the fundus; wherein
the light source (1, 61) comprises a light source (1, 61) for emitting light having coherence; and
the ophthalmic apparatus comprises speckle suppressing means (13, 64) arranged in an optical path of the light projecting optical system, for optically suppressing a speckle noise generated on the retro-illumination image, wherein
the speckle suppressing means (64) serves as a light deflection means for deflecting the measurement light, traveling from the light source (61) to the fundus, with respect to the fundus.

2. The ophthalmic measurement apparatus of claim 1,
**characterized in that**
the measuring optical system (10) further includes drive means (71) for moving an optical member (7) in an optical axis direction to adjust an optical path difference between one portion of light reflected by the fundus and another portion of the light, the other portion of the light being reflected from the cornea or being light from a reference arm of the measuring optical system (10), the optical member (7) being arranged in the optical path,
the light source (1) emits low coherent light,
the light projecting optical system (10a) includes a beam splitter (5) for splitting the light emitted from the light source (1) and projects one portion of the split light onto the fundus,
the light receiving optical system (10b) is configured to guide interference light to the light receiving device (21), the interference light being obtained by interference of the one portion of the light reflected by the fundus and the another portion of the light,
the light receiving device (21) outputs an interference signal based on the interference light received;
the ophthalmic apparatus further comprises control means (80) receiving the interference signal and calculating an ocular axial length of the examinee's eye based on the interference signal output from the light receiving device; and
the speckle suppressing means (13) is arranged in a position out of the optical path of the imaging optical system and in a common optical path thorough which one portion of the light and another portion of the light pass in the measuring optical system.

3. The ophthalmic measurement apparatus according to claim 2, wherein
the speckle suppressing means (13) is a movable speckle suppressing means;
the control means (80) is configured to measure the ocular axial length using the measuring optical system in a state that a movement of the speckle suppressing means is stopped in a first mode in which the ocular axial length is measured by the measuring optical system, and
the control means (80) captures the retro-illumination image using the imaging optical system in a state that the movement of the speckle suppressing means is allowed in a second mode in which the retro-illumination image is captured by the imaging optical system.

4. The ophthalmic measurement apparatus according to claim 2, further comprising:
image processing means (80) for determining a two-dimensional distribution of an opacity portion in a predetermined region by processing the retro-illumination image captured by the imaging device and for specifying a light transmittance region within the predetermined region based on the determined two-dimensional distribution, the predetermined region provided at a certain distance from the corneal apex position and having the corneal apex as a center;
drive means (17) for moving the measuring optical system with respect to the examinee's eye; and
drive control means (80) for controlling the drive means (17) such that an optical axis of the measuring optical system is positioned in the light transmittance region specified by the image processing means.

5. The ophthalmic measurement apparatus according to claim 4, wherein the image processing means partitions the predetermined region into a plurality of partitioned regions, and specifies a partitioned region having a low opacity rate as the light transmittance region by comparing the opacity rates of the partitioned regions using a calculation result of the two-dimensional distribution.

6. The ophthalmic measurement apparatus according to claim 4, wherein the drive control means arranges the optical axis of the measuring optical system in the light transmittance region by controlling the drive means based on an imaging signal output from the imaging device.

7. The ophthalmic measurement apparatus according to claim 1, further comprising:
an apparatus body (8) including the measuring optical system, the imaging optical system, and the speckle suppressing means ;
the measuring optical system being configured to project a measurement target onto the fundus and to receive a light flux thereby to measure an eye refractive power of the examinee's eye;
drive means (17) for moving the apparatus body relative to the examinee's eye; and
control means (80),
wherein, in a retro-illumination image capturing mode in which the retro-illumination image is captured using the imaging optical system, the control means controls the drive means such that the position coordinates of the corneal luminescent spot are substantially the same as those of the outer circumference of the retro-illumination image or are outside thereof, and captures the retro-illumination image by controlling the imaging optical system.

## Patentansprüche

1. Eine ophthalmische Messvorrichtung, die ein Autorefraktometer oder eine Optische-Interferenz-Augenachsenlänge-Messvorrichtung ist, zum Messen eines Auges einer zu untersuchenden Person auf der Basis einer Ausgabe von einem Lichtaufnahmebauelement, die folgende Merkmale aufweist:
ein optisches Messsystem (10, 60) zum Messen des Auges der zu untersuchenden Person durch Projizieren eines Messlichtstroms auf einen Hintergrund des Auges der zu untersuchenden Person und Aufnehmen des Lichts, das dadurch reflektiert wird, wobei das optische Messsystem (10, 60) Folgendes umfasst:
eine Lichtquelle (1, 61);
ein optisches Lichtprojektionssystem (10a, 60a) zum Projizieren des Lichts, das zumindest ein Teil des von der Lichtquelle emittierten Lichts ist, auf einen Hintergrund des Auges der zu untersuchenden Person; und
ein optisches Lichtaufnahmesystem (10b, 60b) zum Leiten von Licht, das das durch den Hintergrund des Auges der zu untersuchenden Person reflektierte Messlicht umfasst, zu dem Lichtaufnahmebauelement (21, 79); und
ein optisches Bilderzeugungssystem (30, 110), das ein Bilderzeugungsbauelement (35, 112) mit einer Bilderzeugungsoberfläche umfasst, die in einer Position angeordnet ist, die im Wesentlichen mit einem vorderen Abschnitt des Auges der zu untersuchenden Person verbunden ist, zum Erfassen eines Retroilluminationsbilds unter Verwendung des Bilderzeugungsbauelements, indem eine Innenpupille von hinten mit dem Messlicht beleuchtet wird, das durch den Augenhintergrund reflektiert wird;
wobei
die Lichtquelle (1, 61) eine Lichtquelle (1, 61) zum Emittieren von Licht mit einer Kohärenz aufweist; und
die ophthalmische Vorrichtung eine Fleckenunterdrückungseinrichtung (13, 64) aufweist, die in einem Strahlengang des optischen Lichtprojektionssystems angeordnet ist, zur optischen Unterdrückung eines Fleckenrauschens, das auf dem Retroilluminationsbild erzeugt wird, wobei
die Fleckenunterdrückungseinrichtung (64) als eine Lichtablenkungseinrichtung zum Ablenken des Messlichts, das sich von der Lichtquelle (61) zu dem Augenhintergrund bewegt, bezüglich des Augenhintergrunds dient.

2. Die ophthalmische Messvorrichtung gemäß Anspruch 1,
**dadurch gekennzeichnet, dass**
das optische Messsystem (10) ferner eine Antriebseinrichtung (71) zum Bewegen eines optischen Bauglieds (7) in Richtung einer optischen Achse umfasst, um eine Strahlengangsdifferenz zwischen einem Teil des Lichts, das durch den Augenhintergrund reflektiert wird, und einem anderen Teil des Lichts einzustellen, wobei der andere Teil des Lichts von der Hornhaut reflektiert wird oder Licht von einem Referenzarm des optischen Messsystems (10) ist, wobei das optische Bauglied (7) in dem Strahlengang angeordnet ist,
die Lichtquelle (1) niedrig kohärentes Licht emittiert,
das optische Lichtprojektionssystem (10a) einen Strahlteiler (5) zum Teilen des Lichts, das von der Lichtquelle (1) emittiert wird, umfasst und einen Teil des geteilten Lichts auf den Augenhintergrund projiziert,
das optische Lichtaufnahmesystem (10b) ausgebildet ist, Interferenzlicht zu dem Lichtaufnahmebauelement (21) zu leiten, wobei das Interferenzlicht durch Interferenz des einen Teils des Lichts, das durch den Augenhintergrund reflektiert wird, und des anderen Teils des Lichts erhalten wird,
das Lichtaufnahmebauelement (21) ein Interferenzsignal auf der Basis des aufgenommenen Interferenzlichts ausgibt;
die ophthalmische Vorrichtung ferner eine Steuereinrichtung (80) aufweist, die das Interferenzsignal empfängt und eine Augenachsenlänge des Auges der zu untersuchenden Person auf der Basis des Interferenzsignals berechnet, das von dem Lichtaufnahmebauelement ausgegeben wird; und
die Fleckenunterdrückungseinrichtung (13) in einer Position außerhalb des Strahlengangs des optischen Bilderzeugungssystems und in einem gemeinsamen Strahlengang, durch den ein Teil des Lichts und ein anderer Teil des Lichts verlaufen, in dem optischen Messsystem angeordnet ist.

3. Die ophthalmische Messvorrichtung gemäß Anspruch 2, bei der
die Fleckenunterdrückungseinrichtung (13) eine bewegbare Fleckenunterdrückungseinrichtung ist;
die Steuereinrichtung (80) ausgebildet ist, die Augenachsenlänge unter Verwendung des optischen Messsystems in einem Zustand zu messen, in dem eine Bewegung der Fleckenunterdrückungseinrichtung gestoppt ist, in einem ersten Modus, in dem die Augenachsenlänge durch das optische Messsystem gemessen wird, und
die Steuereinrichtung (80) das Retroilluminationsbild unter Verwendung des optischen Bilderzeugungssystems in einem Zustand erfasst, in dem die Bewegung der Fleckenunterdrückungseinrichtung zugelassen ist, in einem zweiten Modus, in dem das Retroilluminationsbild durch das optische Bilderzeugungssystem erfasst wird.

4. Die ophthalmische Messvorrichtung gemäß Anspruch 2, die ferner folgende Merkmale aufweist:
eine Bildverarbeitungseinrichtung (80) zum Bestimmen einer zweidimensionalen Verteilung eines Lichtundurchlässigkeitsabschnitts in einer vorbestimmten Region durch Verarbeiten des Retroilluminationsbilds, das durch das Bilderzeugungsbauelement erfasst wird, und zum Angeben einer Lichtdurchlässigkeitsregion innerhalb der vorbestimmten Region auf der Basis der bestimmten zweidimensionalen Verteilung, wobei die vorbestimmte Region in einem bestimmten Abstand von der Hornhautscheitelpunktposition vorgesehen ist und den Hornhautscheitelpunkt als eine Mitte hat;
eine Antriebseinrichtung (17) zum Bewegen des optischen Messsystems bezüglich des Auges der zu untersuchenden Person; und
eine Antriebssteuerungseinrichtung (80) zum Steuern der Antriebseinrichtung (17), so dass eine optische Achse des optischen Messsystems in der Lichtdurchlässigkeitsregion positioniert ist, die durch die Bildverarbeitungseinrichtung angegeben ist.

5. Die ophthalmische Messvorrichtung gemäß Anspruch 4, bei der die Bildverarbeitungseinrichtung die vorbestimmte Region in eine Mehrzahl von partitionierten Regionen partitioniert und eine partitionierte Region mit einer geringen Lichtundurchlässigkeitsrate als die Lichtdurchlässigkeitsregion angibt, durch Vergleichen der Lichtundurchlässigkeitsraten der partitionierten Regionen unter Verwendung eines Berechnungsergebnisses der zweidimensionalen Verteilung.

6. Die ophthalmische Messvorrichtung gemäß Anspruch 4, bei der die Antriebssteuereinrichtung die optische Achse des optischen Messsystems in der Lichtdurchlässigkeitsregion anordnet, durch Steuern der Antriebseinrichtung auf der Basis eines Bilderzeugungssignals, das von dem Bilderzeugungsbauelement ausgegeben wird.

7. Die ophthalmische Messvorrichtung gemäß Anspruch 1, die ferner folgende Merkmale aufweist:
einen Vorrichtungskörper (8), der das optische Messsystem, das optische Bilderzeugungssystem und die Fleckenunterdrückungseinrichtung umfasst;
wobei das optische Messsystem ausgebildet ist, ein Messziel auf den Augenhintergrund zu projizieren und dadurch einen Lichtstrom aufzunehmen, um eine Augenbrechkraft des Auges der zu untersuchenden Person zu messen;
eine Antriebseinrichtung (17) zum Bewegen des Vorrichtungskörpers relativ zu dem Auge der zu untersuchenden Person; und
eine Steuereinrichtung (80),
wobei in einem Retroilluminationsbild-Erfassungsmodus, in dem das Retroilluminationsbild unter Verwendung des optischen Bilderzeugungssystems erfasst wird, die Steuereinrichtung die Antriebseinrichtung derart steuert, dass die Positionskoordinaten des Hornhautleuchtpunkts im Wesentlichen dieselben sind wie diejenigen des Außenumfangs des Retroilluminationsbilds oder sich außerhalb desselben befinden, und das Retroilluminationsbild durch Steuern des optischen Bilderzeugungssystems erfasst.

## Revendications

1. Appareil de mesure ophtalmique, qui est un auto-réfractomètre ou appareil de mesure de longueur axiale oculaire d'interférence optique, pour mesurer l'oeil d'une personne examinée sur base d'une sortie d'un dispositif de réception de lumière, comprenant:
un système optique de mesure (10, 60) destiné à mesurer l'oeil de la personne examinée en projetant un flux de lumière de mesure sur un fond de l'oeil de la personne examinée et à recevoir ainsi la lumière réfléchie, le système optique de mesure (10, 60) comportant:
une source de lumière (1,61);
un système optique de projection de lumière (10a, 60a) destiné à projeter la lumière, qui est au moins une partie de la lumière émise par la source de lumière, sur un fond de l'oeil de la personne examinée; et
un système optique de réception de lumière (10b, 60b) destiné à guider la lumière comportant la lumière de mesure réfléchie par le fond de l'oeil de la personne examinée vers le dispositif de réception de lumière (21, 79); et
un système optique d'imagerie (30, 110), comportant un dispositif d'imagerie (35, 112) présentant une surface d'imagerie disposée en une position sensiblement conjuguée avec un segment antérieur de l'oeil de la personne examinée, destiné à capturer une image de rétro-illumination à l'aide du dispositif d'imagerie en illuminant une pupille intérieure de l'arrière par la lumière de mesure réfléchie par le fond d'oeil;
dans lequel
la source de lumière (1, 61) comprend une source de lumière (1, 61) destinée à émettre une lumière présentant une cohérence; et
l'appareil ophtalmique comprend un moyen de suppression de chatoiement (13, 64) disposé dans un trajet optique du système optique de projection de lumière, destiné à supprimer optiquement un bruit de chatoiement généré sur l'image de rétro-illumination, où
le moyen de suppression de chatoiement (64) sert de moyen de déviation de lumière destiné à dévier la lumière de mesure, qui se déplace de la source de lumière (61) vers le fond de l'oeil, par rapport au fond de l'oeil.

2. Appareil de mesure ophtalmique selon la revendication 1,
**caractérisé par le fait que**
le système optique de mesure (10) comporte par ailleurs un moyen d'entraînement (71) destiné à déplacer un élément optique (7) dans une direction d'axe optique pour ajuster une différence de trajet optique entre une partie de la lumière réfléchie par le fond de l'oeil et une autre partie de la lumière, l'autre partie de la lumière étant réfléchie par la cornée ou étant de la lumière d'un bras de référence du système optique de mesure (10), l'élément optique (7) étant disposé dans le trajet optique,
la source de lumière (1) émet de la lumière peu cohérente,
le système optique de projection de lumière (10a) comporte un diviseur de faisceau (5) destiné à diviser la lumière émise par la source de lumière (1) et projette une partie de la lumière divisée sur le fond de l'oeil,
le système optique de réception de lumière (10b) est configuré pour guider la lumière d'interférence vers le dispositif de réception de lumière (21), la lumière d'interférence étant obtenue par interférence de l'une partie de la lumière réfléchie par le fond de l'oeil et l'autre partie de la lumière,
le dispositif de réception de lumière (21) émet un signal d'interférence sur base de la lumière d'interférence reçue;
l'appareil ophtalmique comprend par ailleurs un moyen de commande (80) recevant le signal d'interférence et calculant une longueur axiale oculaire de l'oeil de la personne examinée sur base du signal d'interférence sorti par le dispositif de réception de lumière; et
le moyen de suppression de chatoiement (13) est disposé en une position hors du trajet optique du système optique d'imagerie et dans un trajet optique commun par lequel une partie de la lumière et une autre partie de la lumière passent dans le système optique de mesure.

3. Appareil de mesure ophtalmique selon la revendication 2, dans lequel
le moyen de suppression de chatoiement (13) est un moyen de suppression de chatoiement mobile;
le moyen de commande (80) est configuré pour mesurer la longueur axiale oculaire à l'aide du système optique de mesure dans un état où un mouvement du moyen de suppression du chatoiement est arrêté dans un premier mode dans lequel la longueur axiale oculaire est mesurée par le système optique de mesure, et
le moyen de commande (80) capture l'image de rétro-illumination à l'aide du système d'imagerie optique dans un état où le mouvement du moyen de suppression de chatoiement est autorisé dans un deuxième mode dans lequel l'image rétro-illumination est captée par le système optique d'imagerie.

4. Appareil de mesure ophtalmique selon la revendication 2, comprenant par ailleurs:
un moyen de traitement d'image (80) destiné à déterminer une distribution bidimensionnelle d'une partie d'opacité dans une région prédéterminée en traitant l'image de rétro-illumination capturée par le dispositif d'imagerie et à spécifier une région de transmission de la lumière dans la région prédéterminée sur base de la distribution bidimensionnelle déterminée, la région prédéterminée étant prévue à une certaine distance de la position de sommet de la cornée et présentant le sommet de la cornée comme centre;
un moyen d'entraînement (17) destiné à déplacer le système optique de mesure par rapport à l'oeil de la personne examinée; et
un moyen de commande d'entraînement (80) destiné à commander le moyen d'entraînement (17) de sorte qu'un axe optique du système optique de mesure soit positionné dans la zone de transmission de lumière spécifiée par le moyen de traitement d'image.

5. Appareil de mesure ophtalmique selon la revendication 4, dans lequel le moyen de traitement d'image divise la région prédéterminée en une pluralité de régions divisées, et spécifie une zone divisée présentant un faible taux d'opacité comme région de transmission de lumière, en comparant les taux d'opacité des régions divisées à l'aide d'un résultat de calcul de la distribution bidimensionnelle.

6. Appareil de mesure ophtalmique selon la revendication 4, dans lequel le moyen de commande d'entraînement dispose l'axe optique du système optique de mesure dans la région de transmission de lumière en commandant le moyen d'entraînement sur base d'un signal d'imagerie sorti par le dispositif d'imagerie.

7. Appareil de mesure ophtalmique selon la revendication 1, comprenant par ailleurs:
un corps d'appareil (8) comportant le système optique de mesure, le système optique d'imagerie et le moyen de suppression de chatoiement;
le système optique de mesure étant configuré pour projeter une cible de mesure sur le fond de l'oeil et pour recevoir un flux de lumière pour mesurer ainsi une puissance de réfraction oculaire de l'oeil de la personne examinée;
un moyen d'entraînement (17) destiné à déplacer le corps d'appareil par rapport à l'oeil de la personne examinée; et
un moyen de commande (80),
dans lequel, dans un mode de capture d'image de rétro-illumination dans lequel l'image de rétro-illumination est captée à l'aide du système optique d'imagerie, le moyen de commande contrôle le moyen d'entraînement de sorte que les coordonnées de position du point luminescent de la cornée soient sensiblement les mêmes que celles de la circonférence extérieure de l'image de rétro-illumination ou se situent à l'extérieur de cette dernière, et capture l'image de rétro-illumination en commandant le système optique d'imagerie.
